# EUROPEAN PATENT SPECIFICATION

(11) **EP 1 882 450 B1**
(45) Date of publication and mention of the grant of the patent: **02.01.2013**
(21) Application number: 06746590.6
(22) Date of filing: 18.05.2006
(51) Int. Cl.: A61B 8/08, A61B 8/14, G06T 7/00, G06T 7/20

(54) **ULTRASONOGRAPHIC DEVICE AND IMAGE PROCESSING METHOD THEREOF**
ULTRASCHALLGERÄT UND BILDBEARBEITUNGSVERFAHREN DAFÜR
DISPOSITIF ECHOGRAPHIQUE ET PROCEDE DE TRAITEMENT D'IMAGE DE CELUI-CI

(30) Priority: 19.05.2005 JP 2005146656
(43) Date of publication of application: 30.01.2008
(73) Proprietor: Hitachi Medical Corporation, Tokyo (JP)
(72) Inventor: CHONO, Tomoaki c/o Hitachi Medical Corporation, Ltd., Tokyo (JP)
(74) Representative: Strehl Schübel-Hopf & Partner
(86) International application number: PCT/JP2006/309902
(87) International publication number: WO 2006/123729

(56) References cited:
- WO-A2-99/49775
- JP-A- 05 123 318
- JP-A- 2000 279 416
- JP-A- 2002 052 026
- JP-A- 2003 061 964
- JP-A- 2004 141 514
- BAMBER J C ET AL: "Adaptive filtering for reduction of speckle in ultrasonic pulse-echo images" ULTRASONICS, IPC SCIENCE AND TECHNOLOGY PRESS LTD. GUILDFORD, GB LNKD- DOI:10.1016/0041-624X(86)90072-7, vol. 24, no. 1, 1 January 1986 (1986-01-01), pages 41-44, XP025703369 ISSN: 0041-624X [retrieved on 1986-01-01]
- CHEN C W ET AL: "A Novel Algorithm for Computer-Assisted Measurement of Cervical Length From Transvaginal Ultrasound Images" IEEE TRANSACTIONS ON INFORMATION TECHNOLOGY IN BIOMEDICINE, IEEE SERVICE CENTER, LOS ALAMITOS, CA, US LNKD- DOI:10.1109/TITB.2004.828891, vol. 8, no. 3, 1 September 2004 (2004-09-01), pages 333-342, XP011118225 ISSN: 1089-7771

## Description

### Technical Field

The present invention relates to an ultrasonic diagnostic apparatus and image processing method thereof, particularly the ones capable of obtaining useful ultrasonic images considering the shape and size of speckles.

### Background Art

In ultrasonic images obtained by an ultrasonic diagnostic apparatus, noise referred to as speckle noise is mixed in. It has been considered that speckle noise appears when scattered waves caused by a sufficiently small reflector group compared to the wavelength of ultrasonic waves are generated in a variety of phases and interfere with each other.
Generally speckle noise has considered to be reduced since it is unnecessary for image diagnosis. For example, a circuit for determining and eliminating speckle noise is provided in the conventional technique disclosed in Patent Document 1.
Patent Document 1: JP-A-H9-94248

However, the present inventers viewed speckles in ultrasonic images as not necessarily unuseful information upon diagnosis of an object to be examined. That is to consider that images more valuable for diagnosis can be obtained without comprising an eliminating circuit for speckle noise, by executing filtering process in accordance with shape and size of speckles appearing on the images.

The scientific article "Adaptive filtering for reduction of speckle in ultrasonic pulse-echo images" by J. C. Bamber and C. Daft, Ultrasonics, IPC Science and Technology Press Ltd., Guildford, UK, vol. 24, no. 1, January 1, 1986, pp. 41-44, XP025703369, ISSN: 0041-624X, and the Japanese patent application JP 2003 061964 A describe speckle filtering processes in which the spatial bandwidth of smoothing filters and the grade of the smoothing matrix, respectively, are controlled by similarity. In the article *"*A novel algorithm for computer-assisted measurement of cervical length from transvaginal ultrasound images" by M. Wu et al., IEEE Transactions on Information Technology in Biomedicine, IEEE Service Center, Los Alamitos, US, vol. 8, no. 3, September 1, 2004, pp. 333-342, XP011118225, ISSN: 1089-7771, speckle suppression is based on the average distance between adjacent speckles. WO 99/49775 A2 discloses an echocardiography workstation related to the ultrasonic diagnostic apparatus of the present invention.

### Disclosure of the Invention

The objective of the present invention is to provide an ultrasonic diagnostic apparatus and image processing method thereof capable of obtaining more dynamic ultrasonic images by executing filtering process taking shape and size of speckles into consideration.
More concretely, it is to provide an ultrasonic diagnostic apparatus and image processing method thereof capable of contributing to a proper diagnosis of a heart lesion, particularly capable of capturing dynamic images of a plurality of cardiac regions, that is four-chambers such as a left ventricle, cardiac muscle, left atrium, right ventricle and right atrium, so as to evaluate the function of those regions.

In order to achieve the above-mentioned objective, an ultrasonic diagnostic apparatus of the present invention comprising means for imaging moving-images of an object to be examined by transmitting/receiving ultrasonic waves to/from the obj ect is defined in claim 1.

Also, an ultrasonic image processing method of the present invention is defined in claim 11.

### Brief Description of the Diagrams

Fig. 1 is a system configuration diagram of an ultrasonic diagnostic apparatus related to embodiment 1 of the present invention.
Fig. 2 is an operating procedure of ultrasonic diagnostic apparatus 1 related to embodiment 1 of the present invention.
Fig. 3 shows a state of setting a window in a direction of ultrasonic beams on an ultrasonic image (B-mode image).
Fig. 4 shows an ideal profile of a speckle.
Fig. 5 shows a speckle displayed in an oval figure.
Fig. 6 shows a case in which the interval of the speckles is narrow and the contrast is being saturated.
Fig. 7 shows an example of the characteristics of 2-dimensional Gaussian filtering.
Fig. 8 shows a condition in which manual tracing is completed.
Fig. 9 shows the result of correcting irregularity or dispersion of intervals of contour points 83 ~ 88 by step 26.
Fig. 10 is an illustration of the Simpson method.
Fig. 11 is a graph showing how the respective parameters vary (time variation) along with updating of the frame.
Fig. 12 shows a display example in embodiment 2.
Fig. 13 shows a display example in embodiment 3.
Fig. 14 shows another display example on a display unit.

### Best Mode to Carry Out the Invention

Hereinafter, the desirable embodiments of the present invention will be described referring to the diagrams.

### Embodiment 1

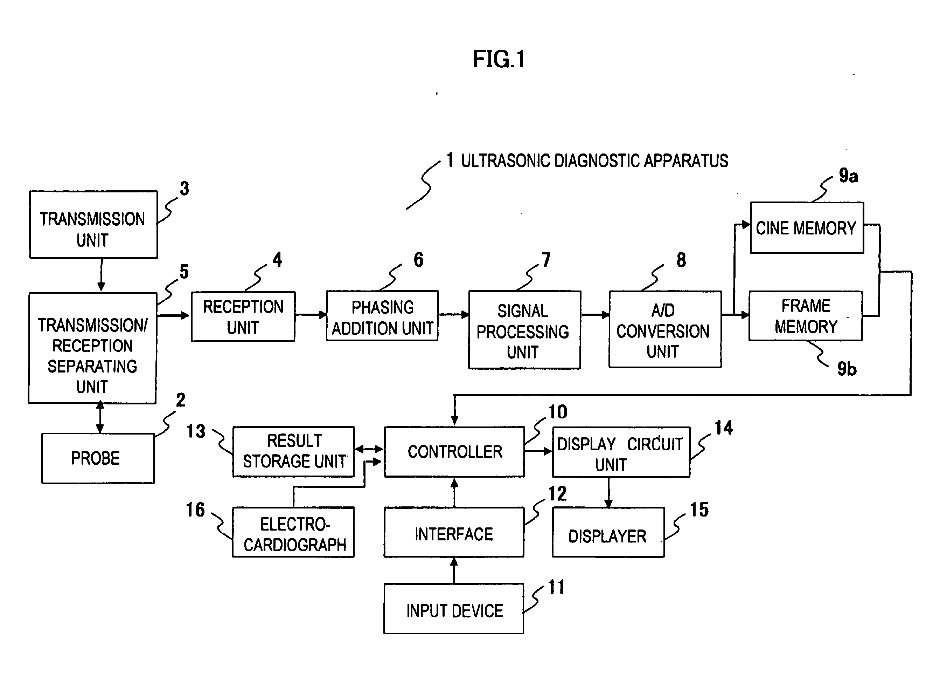

Fig. 1 is a system configuration diagram of an ultrasonic diagnostic apparatus related to embodiment 1 of the present invention.
In Fig. 1, the ultrasonic diagnostic apparatus 1 related to embodiment 1 of the present invention is an apparatus for measuring cardiac function using ultrasonic waves, and has configuration comprising heretofore known ultrasonic diagnostic apparatus as at least a part of the apparatus.

Ultrasonic diagnostic apparatus 1 is configured comprising probe 2, transmission unit 3, reception unit 4, transmission/reception separating unit 5, phasing addition unit 6, signal processing unit 7, A/D conversion unit 8, frame memory 9a, cine memory 9b, controller 10, input device 11, interface 12, result storage unit 13, display circuit unit 14, display unit 15 and electrocardiograph 16. In Fig. 1, only major functions are illustrated. Hereinafter, each configuration shown in Fig. 1 will be described.

Probe 2 is configured to transmit ultrasonic waves to a diagnostic region (here, a heart) and to receive the reflected waves. Inside of probe 2, a plurality of transducers not shown in the diagram is provided which is a generation source (transmission source) of ultrasonic waves and capable of receiving the reflected waves. Transmission unit 3 is for generating transmission pulse signals for transmitting ultrasonic waves by driving probe 2. On the other hand, reception unit 4 is for receiving the echo signals that are received by probe 2 and converted into electrical signals.

Transmission/reception separating unit 5 transmits transmission pulse signals from transmission unit 3 to probe 2 upon transmission, and transmits the echo signals from probe 2 to reception 4 upon reception. Phasing addition unit 6 is for performing phasing addition on a plurality of echo signals from reception unit 4 and generating reception beam signals.

Signal processing unit 7, A/D converter 8, frame memory 9a and cine memory 9b operate as a signal-processing unit for obtaining grayscale tomographic image (black and white tomographic image) of a diagnostic region based on the reception beam signals. More specifically, signals processing unit 7 inputs reception beam signals from phasing addition unit 6, and performs signal processing such as gain compensation, log compression, detection, edge enhancement and filtering process. A/D converter 8 is for converting signals outputted from signal processing unit 7 into digital signals. Frame memory 9a is configured to store digital reception beam signals outputted from the A/D converter by the image frame unit. Also, cine memory 9b is for storing a plurality of image frames that are consecutively imaged. The images for being stored in frame memory 9a and cine memory 9b are configured to correspond with phase information of ECG wave pattern measured in electrocardiograph 16.

The above-mentioned tomographic frame data stored in frame memory 9a is read out by TV synchronism based on control signals from controller 10. Controller 10 is for performing a variety of processes such as controlling the respective components based on a control program, converting tomographic frame data being read out from cine memory 9a into ultrasonic tomographic data, generating data of the contour points or contour lines to be described below, controlling output operation to display unit 15, performing after-described volume calculation related to measurement in cardiac function, predetermined calculation of distance, correction, smoothing process and tissue tracking.

Controller 10 is provided with the same functions as a so-called microcomputer. It also provides after-mentioned functions such as calculation means, means for outputting operation result, smoothing process means and tissue-tracking means.

Input device 11 is connected to controller 10 via interface 12. A mouse or trackball can be cited as an example for input device 11. Input device 11 is provided for the purpose of manually tracing each contour of a left ventricle, cardiac muscle and left atrium of a heart on an ultrasonic image by a technician (operator) while referring to the ultrasonic image displayed on display unit 15. Input device 11 and controller 10 function as tracing means and correction means to be described later.

Result storage unit 13 is provided with function as a memory for storing after-mentioned information such as coordinate data of contour points or result of calculation performed in controller 10. Information such as coordinate data or calculation result stored in result storage unit 13 is set to be read out based on controlling signals from controller 10.

Display circuit unit 14 is configured to operate based on control signals related to the outputting from controller 10. Display circuit unit 14 converts ultrasonic tomographic data from controller 10 or data of the after-mentioned contour points and the respective contour lines into analogue signals, and generates picture signals for display. Though not shown in the diagram, a device such as D/A converter or picture signal converting circuit is provided in display circuit 14. Display unit 15 is for inputting picture signals outputted from display circuit unit 14 and displaying ultrasonic images. A TV monitor, for example, is used for display unit 15.

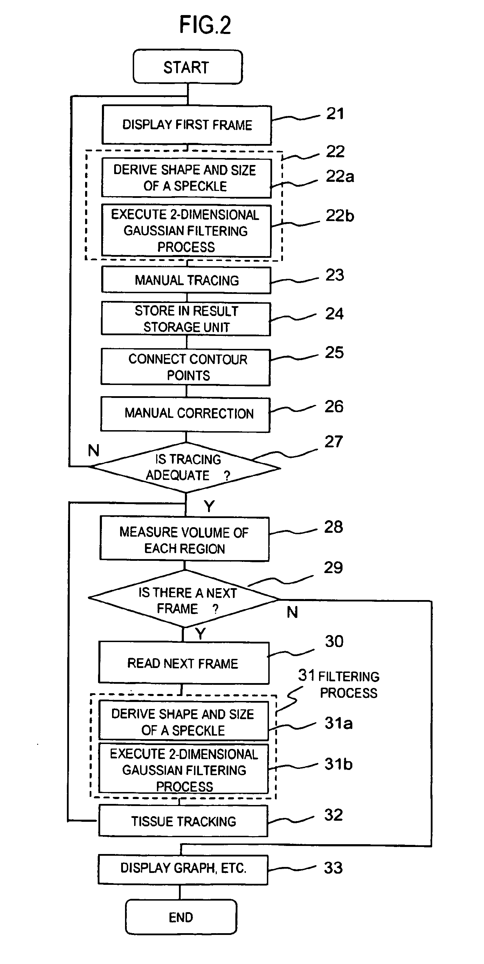

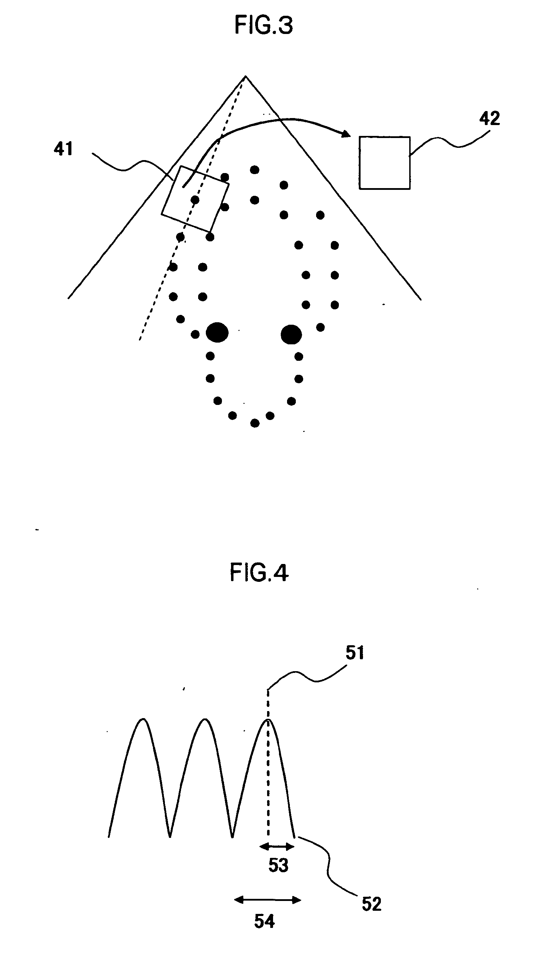

Next, processing procedure of ultrasonic diagnostic apparatus 1 related to embodiment 1 of the present invention will be described referring to a flow chart in Fig. 2. Fig. 2 is the flow chart showing the process in controller 10. Procedures for a various input operations to be carried out by a user using input device 11 and display unit 15 will also be included in the process to be explained below. Also, the explanation of the respective steps of the flow chart in Fig. 2 will be described referring also to Figs. 3 ~ 11.

### (Step 21)

The image of the first frame is read out from cine memory 9b based on input signals inputted by an operator using input device 11, and the first frame of a moving image of ultrasonic waves is displayed on display unit 15.

### (Step 22)

Filtering process is performed, by a method to be described later, on the first frame image displayed in step 21 to improve image quality.

Hereinafter, the filtering process to be performed in the present step will be described in detail. Here, the filtering process for performing appropriate calculation (differentiation, etc.) in step 32 upon process such as tissue tracking will be described in detail. This filtering process is formed by step 22a and step 22b.
Generally, in order to properly perform differential operation, smoothing filter of image density is performed on image data. One of the smoothing filters is a 2-dimensional Gaussian filter. In an ultrasonic image, since there is a difference in a resolution between the transmitting/receiving direction of ultrasonic beams and scanning direction (direction to intersect with the transmitting/receiving direction), it is necessary to perform a 2-dimensional Gaussian filter in accordance with the difference of resolution. More specifically, while there is irregularity of density referred to as speckles on ultrasonic images (for example, refer to JP-A-H7-51270), these speckles are not complete circle shapes but elliptical shapes (major axis direction and minor axis direction are respectively the scanning direction or transmitting direction of ultrasonic beams). The present inventor took into consideration the fact that the speckles are elliptical shape, and invented a method to perform a 2-dimensional Gaussian filter in anisotropic manner.

Hereinafter steps 22a and 22b will be described.

### (Step 22a)

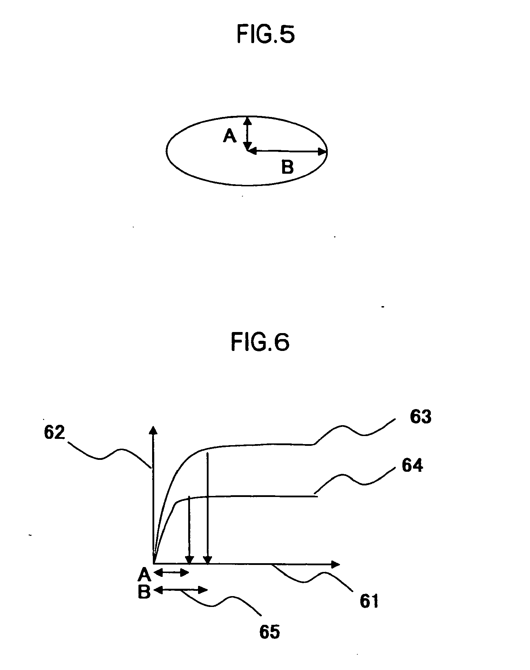

Fig. 3 is a state of setting window 41 on an ultrasonic image (B-mode) in ultrasonic beam direction. First, average size and/or shape of a speckle within the window are obtained from image data within window 41. Concrete procedure is to obtain feature quantity of contrast by taking out the pixel values in the window as it is, performing affine transformation so that transmitting direction of the beams is directed vertical on an image, and calculating a density cooccurrence matrix in horizontal direction and vertical direction (for example, refer to JP-A-H5-123318) within the window indicated as 42. In a case that size and/or shape of a speckle is ideal, as shown in Fig. 4, distance (53) between pixel position (51) wherein the contrast is the highest and pixel position (52) wherein the contrast is the lowest becomes a half of the size of the speckle (54). Therefore, the size of the speckle (minor axis A and major axis B) in the case of being approximated by elliptical shape (Fig. 5) is obtained by calculating distance (53) between the pixel position (51) wherein the contrast is the highest and pixel position (52) wherein the contrast is the lowest in horizontal direction and vertical direction.

Or, there are cases that the profile on a line segment (on the line segment within window 42 in Fig. 3) on an image is not necessarily ideal as shown in Fig. 4. For example, Fig. 6 is the result of calculating the density cooccurrence matrix, 61 is distance between the pixels, 62 is the contrast, 63 is the contrast in lateral direction, 64 is the contrast in lengthwise direction, and distance 65 indicated in A and B is minor axis A and maj or axis B of the speckle. In Fig. 6, interval between the speckles is narrow, and the contrast feature quantity of the density cooccurrence matrix is being saturated. In the case such as Fig. 6, distance until the pixel value reaches the maximum (A and B in Fig. 6) is detected and set as the size of the speckle (minor axis A and major axis B).

### (Step 22b)

In the present step, using the size of the speckle obtained in step 22a (minor axis A and major axis B), the 2-diemensional Gaussian filter is applied in accordance with the feature thereof.

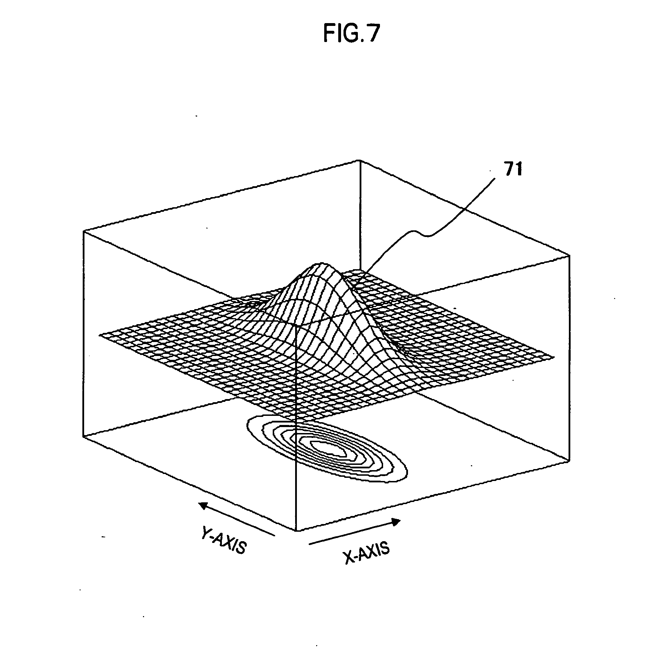

Fig. 7 is an example of characteristics of 2-dimentional Gaussian filter (71). While 2-dimensional Gaussian filter is for using the function characterized in having normal distribution at any cross-section in X-axis direction and Y-axis direction as shown in Fig. 7, when the function shown in Fig. 7 is cut in XY-plane, the cross-sectional surface turns out to be an elliptical shape which is the same as the speckle obtained in step 22a. In the present step, length of minor axis A and major axis B in step 22a is adjusted as standard deviation in X-axis direction and Y-axis direction of 2-dimensional Gaussian filter shown in Fig. 7 and the smoothing process is optimized.

In this regard, however, as for how to adjust standard deviation difference σ_{A} and σ_{B} in X-direction and Y-direction of 2-dimensional Gaussian filter with respect to minor axis A and major axis B obtained in step 22a, it is important to set them somewhat bigger than the length of minor axis A and major axis B in order not to generate unnecessary noise. Also, it is necessary to adjust standard deviation difference appropriately, for there will be a problem of losing the characteristic of an image due to too much smoothing when standard deviation σ_{A} and σ_{B} is set too high.
By using the above-mentioned filtering process (steps 22a and 22b), the left atrium (or right atrium) of a heart can be displayed.

### (Step 23)

An operator starts manual tracing of the four-chambers of the heart using input device 11 formed by a mouse or trackball while observing the ultrasonic image of the first frame displayed on display unit 15. In the present embodiment, since the left atrium could be displayed with clarity in steps 22a and 22b, manual tracing of the left atrium can be easily implemented. Here, manual tracing means that the operator traces the left ventricle, cardiac muscle and the contour of the left atrium (more concretely, inner membrane of the left ventricle, outer membrane of the left ventricle and the contour of the left atrium) by tracing the points (contour points) on an ultrasonic image. Also, in the present embodiment, a pair of valve ring (joining of the left ventricle and the left atrium) is set down as an intersection upon manual tracing of the left ventricle and left atrium.

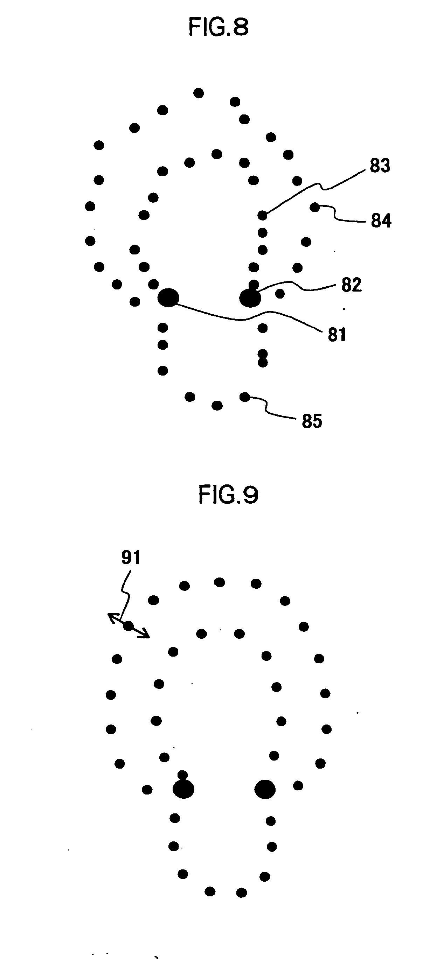

An example of a concrete procedure for manual tracing here is to place a contour point at the position of one valve ring (for example, 81 in Fig. 8), and a plurality of contour points are placed in sequence along the inner membrane of the left ventricle from this contour point. A contour point is placed at the position of the other valve ring (for example, 82 in Fig. 8) after the plurality of contour points are placed along the inner membrane of the left ventricle. In the same manner, a contour point is placed at the position of one valve ring, and a plurality of contour points are placed in sequence along the outer membrane of the left ventricle from this contour point. After the plurality of contour points are placed along the outer membrane of the left ventricle, a contour point is placed at the position of the other valve ring. Further, a contour point is placed at the position of one valve ring, and a plurality of contour points are placed in sequence along the left atrium from this contour point. After the plurality of contour points are placed along the left atrium, a contour point is placed at the other position of the valve ring.

The procedure of manual tracing illustrated here is mere an example, and the manual tracing can be implemented from any contour point. Manual tracing (placement of a contour point) can also be implemented in either clockwise or counterclockwise direction on an image. Also, only the left atrium can be manual traced and the left ventricle and cardiac muscle can be traced automatically using a conventional technique (for example, the technique disclosed in JP-A-H8-206117).

### (Step 24)

The contour point placed by inputting using input device 11 in step 23 is displayed on display unit 15 being superimposed over an ultrasonic image, and stored in result storage unit 13.
Fig. 8 shows a condition that the manual tracing is completed, and a plurality of contour points have been placed. In Fig. 8, 81 and 82 indicate the position of a pair of valve rings. Also, 83 indicates a contour line of the inner membrane of the left ventricle formed by a plurality of contour points, 84 indicates a contour line of the outer membrane of the left ventricle, and 85 a contour line of the left atrium. As is clear from Fig. 8, when the contour points are placed by manual-tracing, contour lines 83 ~ 85 have much irregularity and intervals of the contour points vary widely.

### (Step 25)

On the basis of control by controller 10, automatic correction is performed on irregularity of the manually traced three contour lines 83 ~ 85 or variation of the intervals. To be more precise, for example, the contours may be rearranged to be the number and intervals set in advance by performing a fitting such as a spline curve. Fig. 9 is a result of performing correction in step 25 on the irregularity of contour lines 83 - 85 or interval variation, the contour lines are smoother.

### (Step 26)

In the case that the operator recognizes the contour point wherein the fitting is falsely implemented as a result of automatic correction in step 25, manual correction is to be carried out using input device 11. Manual correction is implemented clicking or dragging the respective contour points. Coordinate data of the respective contour points after manual correction is stored again in result storage unit 13.

Meanwhile, at the time of the above-mentioned manual tracing or the correction of the manual-traced contour points, there is a possibility that the position of the valve rings are slightly displaced by the corrected contour lines 83 ~ 85. In such a case, they may be standardized by the position of the valve rings determined by any one contour line, or standardized by obtaining the average coordinate of the positional coordinate of the valve rings from the plurality of contour lines and setting them as the standardized position of the valve rings. By doing so, the left ventricle and the left atrium can be connected by one line, and blood flow volume flowing between the left ventricle and the left atrium can be measured without omission. Also, since the region enclosed by the inner membrane of the left ventricle and the outer membrane of the left ventricle is the cardiac muscle, regions such as an area of the cardiac muscle region can be measured without omission.

### (Step 27)

Whether the manual tracing of the first frame is properly carried out or not is determined, and if it is determined to be properly executed step 28 is to proceed. If it is determined not properly executed, step 21 is to proceed.

### (Step 28)

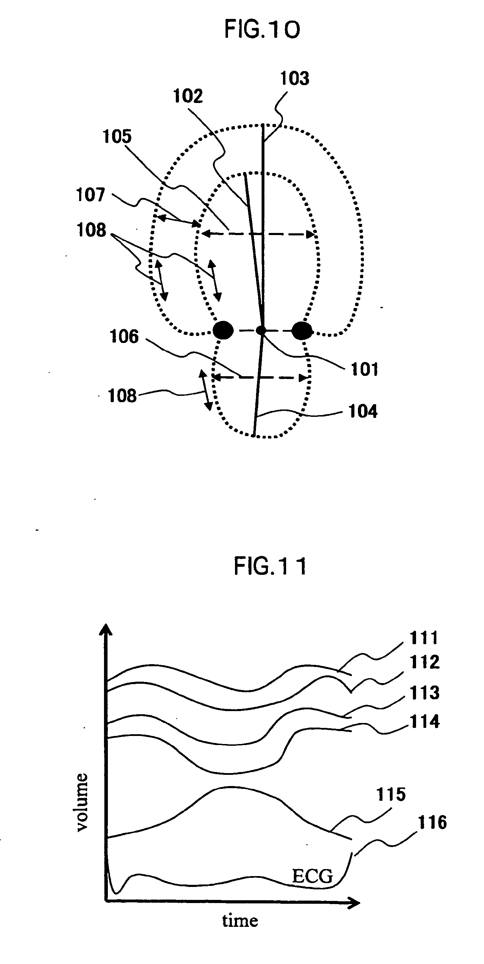

Based on the contour line obtained up to step 26, volume and size (distance, etc.) of the respective regions of the heart is measured. For example, the Simpson method is used for obtaining the volume in the present embodiment, and the concrete procedure will be described here referring to Fig. 10. First, the Simpson method is applied by obtaining midpoint 101 between the valve rings, searching the farthest point from the obtained midpoint on each contour line of the inner membrane of the left ventricle, outer membrane of the left ventricle and the left atrium, and obtaining axes 102, 103 and 104 by connecting the obtained farthest point and midpoint 101. The method for performing quadrature of organs using the Simpson method is disclosed, for example, in JP-A-H7-289545. Using such method disclosed in JP-A-H7-289545, each volume of the inner membrane of the left ventricle, outer membrane of the left ventricle, the left atrium, sum of the left ventricle and the left atrium, and the cardiac muscle (difference between the outer membrane volume of the left ventricle and the inner membrane volume of the left ventricle) is calculated. Also, length of axis 102 (length of the line for connecting the point that intersects with the outer membrane of the left ventricle at the farthest upper side from midpoint 101 on axis 102 and the point that intersects with the left atrium in the case of extending axis 102 toward the farthest bottom side from point 101 on axis 102), distance between the walls of the left ventricle and the left atrium (widths 105 and 106 in direction of the line segment connecting a pair of valve rings of the contour line forming the inner membrane of the left ventricle and the left atrium), and distance 107 between the inner membrane and the outer membrane of the cardiac muscle are calculated. Distance 108 between the contour points in the contour line direction is also calculated.

### (Step 29)

Whether there is a next frame or not is determined. When there is a next frame, step 30 is to proceed. When there is not, step 33 is to proceed.

### (Step 30)

Controller 10 reads out image data of the next frame from cine memory 9b, and stores it in result storage unit 13.

### (Step 31)

Filtering process is performed on the second frame image displayed in step 30 in the same manner as step 22 for improving the image quality. Step 31 is formed with step 31a and step 31b, and the same process as step 22a and step 22b is performed respectively.

### (Step 32)

In the present step, variation of the contour line of the respective organs generated upon moving from the first frame to the second frame (or, from the n-th frame to the n+1-th frame in accordance with the readout of the frame carried out one after another in step 30) is automatically tracked. Here, the tracking of the variation (movement) of the contour line of the respective organs is referred to as the tissue-tracking process. For the concrete method of the tissue tracking process in the present embodiment, an algorithm with high robustness is used to make it applicable even for the case of having low image quality. For example, the optical flow method can be used, and the block matching method, gradient method and particle tracking method are applicable. In gradient method, a velocity vector is analytically obtained by concretely using gradient of the image density. Since the access to the image is only calculation of differentiation, velocity vectors can be obtained with high speed. Particularly in a membrane part, tissue tracking can be stably carried out since large enough differential value can be obtained. When tissue tracking is completed, step 28 proceeds, and each volume of the inner membrane of the left ventricle, outer membrane of the left ventricle, left atrium, sum of the left ventricle and left atrium, and cardiac muscle (difference between the outer membrane volume of the left ventricle and the inner membrane volume of the left ventricle), length of 102, distance between the walls of the left ventricle and left atrium, distance between the inner membrane and outer membrane of cardiac muscle, distance between the contour points in contour line direction are calculated (hereinafter, these values to be obtained in step 28 is referred to as parameter) with respect to the second frame (n+1-th frame).

### (Step 33)

When processing in all the frames is completed, variation of each parameter along with updating of the frames (time variation) is displayed on display unit 15 in graph form. In this graph display, time or numbering of the frames is indicated in lateral axis and the calculated values of each parameter are indicated in vertical axis, and displayed, for example, as shown in Fig. 11.

In Fig. 11, displays time variation of volume of the inner membrane of the left ventricle, outer membrane of the left ventricle, left atrium, sum of the left ventricle and left atrium, cardiac muscle (volume of the outer membrane of the left ventricle minus volume of the inner membrane of the left ventricle), which makes it possible to perform diagnosis while referring to the volume variation of the respective regions in a heart including the left ventricle and ECG (electrocardiograph) on a reciprocal basis (in Fig. 11, the first line from the top indicates the outer membrane of the left ventricle, the next line indicates the sum of the left ventricle and the left atrium, the next line indicates the cardiac muscle, the next line indicates the inner membrane of the left ventricle, the next line indicates the left atrium, and the bottom line indicates the ECG (electrocardiograph). Furthermore, it is possible to graphically display the length of the respective axes of the inner membrane of the left ventricle, outer membrane of the left ventricle and left atrium (102, 103 and 104 in Fig. 10), distances 105 and 106 between the walls of the left ventricle and left atrium, distance 107 between the inner membrane and the outer membrane of the cardiac muscle, and distance 108 between the contour points in contour line direction. Volume, shaft length and distance between walls are an important index for evaluating cardiac function, and is deeply related to the kinematic performance of the cardiac muscle of a left ventricle or the membrane of a left atrium. By the graphical display as mentioned above, it is possible to observe relationship between the time phase and abnormal cardiac function and the difference of cardiac function between the left ventricle and the left atrium.

In accordance with the embodiment above, in the first piece of the moving images formed by a plurality of frames consecutively obtained in terms of time, when the contour line of the respective organs is determined by a method such as manual tracing and the contour line of the respective organs is further tracked by tissue tracking with respect to the plurality of frames continued into the first piece, since the filtering process of the respective image data is performed considering the size and shape of the speckle distinctively appearing on the ultrasonic image, contours of the regions such as the left atrium which have been unclear when obtained by conventional methods are made possible to be tracked. Also, it is possible to provide an ultrasonic diagnostic apparatus and method capable of obtaining various parameters for diagnosis of an object based on the contour line of the respective organs of each frame and displaying the temporal variation thereof.

### Embodiment 2

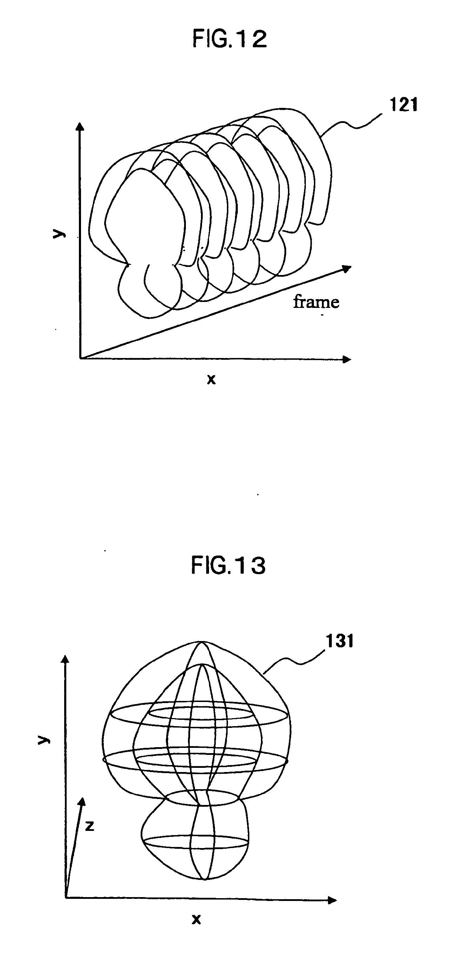

The present embodiment is another display example to be displayed on display unit 15 in the present invention. As seen in Fig. 12, the cross sections of the ventricle, left atrium and cardiac muscle (121) can be displayed 3-dimensionally by lining them up in time-series. By doing so, variation of the heart shape can be visually observed.

### Embodiment 3

The present embodiment is another display example to be displayed on display unit 15 in the present invention. As seen in Fig. 13, by segmentalizing the ventricle, left atrium and cardiac muscle into several parts, 3-dimensionally displaying them (131) and consecutively displaying the frames, it is possible to visualize the temporal variation of the 3-dimensionally displayed ventricle, left atrium and cardiac muscle.

The present invention does not have to be limited to the above-mentioned embodiments, and various changes may be made without departing from the scope of the invention. For example, the filtering process for performing on the respective frames in the above-mentioned step 22 and step 31 do not have to be performed after step 21 and step 30, and can be performed with respect to all of the frames at once before step 21.
Also, the 2-dimensional Gaussian filter to be performed in steps 22b and 31b does not necessarily have to be the filtering process using Gaussian function, and other functions may be used instead.

Also, the present invention can be applied to observe not only a moving state of a heart, but also other organs. For example, it can be used for observing pulse of carotid artery in a neck region. The present invention is also applicable to usual ultrasonic diagnostic apparatus and method, since it is considered effective in improving image quality not only for moving organs but also for regular imaging by ultrasonic waves, by performing smoothing process considering the size and/or shape of a speckle.

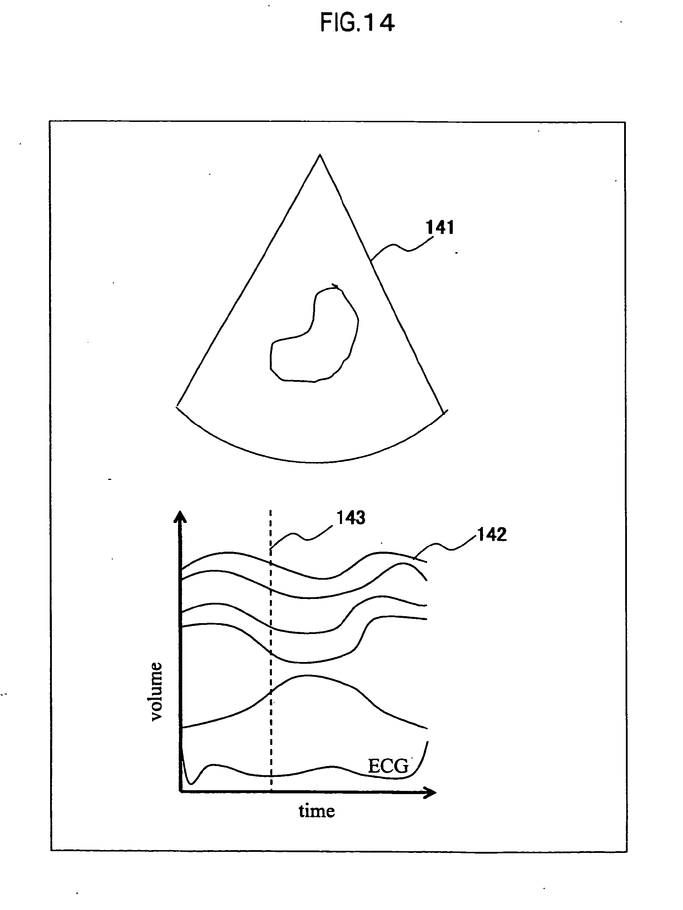

Also, size and/or shape of a speckle can be different by location even within the same frame data of one frame, smoothing process by Gaussian filter may be varied by making it depend on the variation of the size and/or shape by location.
Also, display example shown in Figs. 11 ~ 13 does not have to be displayed individually on display unit 15, and may be displayed juxtaposed to a B-mode image. For example, when the B-mode image (141) and the display example of Fig. 11 (142) are combined, it will be displayed as seen in Fig. 14. In Fig. 14, the line denoted by 143 indicates which timing the upper B-mode image in Fig. 14 belongs to in the time axis expressed by the lateral axis in the lower display example.

## Claims

1. An ultrasonic diagnostic apparatus comprising means for transmitting/receiving ultrasonic waves to/from an object to be examined and imaging a moving object with moving images, comprising:
speckle measuring means, with respect to each frame of the moving images, for measuring a size and/or shape of a speckle appearing on each frame; and
smoothing means for performing a smoothing process on image data of each frame in accordance with the measured size and/or shape of the speckle;
wherein the speckle measuring means are adapted to measure the size and/or shape of the speckle by approximating the speckle by an ellipse with a major axis and a minor axis and measuring its size and/or shape by obtaining the major and minor axes of the approximating ellipse; and
wherein the smoothing means are adapted to perform the smoothing process by a Gaussian filtering process using a 2-dimensional Gaussian filter function and to configure the Gaussian filter function such that its standard deviations in the directions of the two orthogonal axes is based on the lengths of the major axis and the minor axis of the ellipse approximating the size and/or shape of the speckle as measured by the speckle measuring means.

2. The ultrasonic diagnostic apparatus according to claim 1, wherein the speckle measuring means are adapted to measure the size and/or shape of the speckle based on the result of an application of a density cooccurrence matrix performed on image data of the respective frames of the moving image.

3. The ultrasonic diagnostic apparatus according to claim 1, comprising:
parameter measuring means for measuring parameter representing the shape of the moving region based on the contour of the respective regions in each frame of the moving image; and
display means (15) for displaying temporal variation of the parameter.

4. The ultrasonic diagnostic apparatus according to claim 1, wherein:
the moving region is the heart of an object to be examined; and
the contour is a contour of an inner membrane of the left ventricle, outer membrane of the left ventricle, left atrium, inner membrane of the right ventricle, outer membrane of the right ventricle and right atrium of the heart.

5. The ultrasonic diagnostic apparatus according to claim 4, comprising
means for adjusting the respective contours so that each pair of valve rings become a joining point of an inner membrane of a left ventricle, outer membrane of the left ventricle and a left atrium, or of the inner membrane of a right ventricle, outer membrane of the right ventricle and a right atrium of the heart, respectively.

6. The ultrasonic diagnostic apparatus according to claim 3, wherein the parameter is volume or axis length of four chambers of the heart, distance between the membranes by which the heart is formed, or thickness of cardiac muscle.

7. The ultrasonic diagnostic apparatus according to claim 3, wherein the measuring means are adapted to obtain parameters using the Simpson method.

8. The ultrasonic diagnostic apparatus according to claim 1, comprising
means (15) for displaying temporal variation of the contour by 3-dimensionally arraying the cross-sections of the moving region.

9. The ultrasonic diagnostic apparatus according to claim 1, comprising
means (15) for displaying temporal variation of the contour by temporally varying a 3-dimensional image of the contour of the moving region.

10. An ultrasonic image processing method comprising:
1) a step for transmitting/receiving ultrasonic waves to/from an object and imaging a moving object with moving images,
2) a step (22a, 31 a), with respect to each frame of the moving image, for measuring a size and/or shape of a speckle appearing on each frame; and
3) a step (22b, 31 b) for performing a smoothing process on image data of each frame in accordance with the measured size and/or shape of the speckle;
wherein in step 2) the size and/or shape of the speckle is measured by approximating the speckle by an ellipse with a major axis and a minor axis and measuring its size and/or shape by obtaining the major and minor axes of the approximating ellipse; and
wherein in step 3) the smoothing process is performed by a Gaussian filtering process using a 2-dimensional Gaussian filter function configured such that its standard deviations in the two orthogonal axes is based on the lengths of the major axis and the minor axis of the ellipse approximating the size and/or shape of the speckle as measured by the speckle measuring means.

11. The ultrasonic image processing method according to claim 10, comprising:
4) a step for extracting a contour of the moving region with respect to an arbitrary frame of the moving image; and
5) a step for detecting movement of the contour with respect to another frame of the moving image.

12. The ultrasonic image processing method according to claim 10, wherein in step 2), the size and/or shape of the speckle is detected by applying a density cooccurrence matrix on image data in each frame.

13. The ultrasonic image processing method according to claim 10, wherein the contour is a contour of an inner membrane of a left ventricle, outer membrane of the left ventricle, left atrium, inner membrane of a right ventricle, outer membrane of the right ventricle, and right atrium of a heart.

14. The ultrasonic image processing method according to claim 13, wherein step 2) comprises:
6) a step (23) for inputting contour points of an inner membrane of a left ventricle, outer membrane of the left ventricle and left atrium of a heart on one frame of the moving image using an input means;
7) a step for adjusting the contour points to make a portion at which contour points of the inner membrane of the left ventricle, outer membrane of the left ventricle and left atrium of the heart are joined at a valve ring;
8) a step (25) for deriving a contour line by smoothly connecting the contour points; and
9) a step (26) for correcting the derivation of the contour line.

15. The ultrasonic image processing method according to claim 11, further comprising:
10) a step for detecting how the contour derived as a contour line in the step 9) moves in each frame of the moving image;
11) a step for calculating parameter related to the movement of the moving region corresponding to the movement of the contour; and
12) a step for displaying temporal variation of the parameter thereof.

16. The ultrasonic image processing method according to claim 11, comprising:
13) a step for performing signal processing for appropriately displaying movement of the contour; and
14) a step for displaying the movement of the contour based on the result obtained by the signal processing.

17. The ultrasonic image processing method according to claim 16 wherein temporal variation of the contour, in step 14), is displayed by 3-dimensionally arrayed cross-sections of the moving region.

18. The ultrasonic image processing method according to claim 16 , wherein temporal variation of the contour, in step 14), is displayed by temporally varying the 3-dimensional image of a contour of the moving image.

## Patentansprüche

1. Ultraschalldiagnosevorrichtung, umfassend eine Einrichtung zum Übertragen/Empfangen von Ultraschallwellen an/von einem zu untersuchenden Objekt und zum Abbilden eines sich bewegenden Objekts mit Bewegtbildern, umfassend:
eine Fleckmesseinrichtung zum Messen, bezüglich jedes Frames der Bewegtbilder, einer Größe und/oder einer Form eines auf jedem Frame erscheinenden Flecks und
eine Glättungseinrichtung zum Ausführung eines Glättungsvorgangs an den Bilddaten jedes Frames gemäß der gemessenen Größe und/oder Form des Flecks;
wobei die Fleckmesseinrichtung dazu ausgelegt ist, die Größe und/oder die Form des Flecks durch Approximieren des Flecks mit einer Ellipse mit einer Hauptachse und einer Nebenachse und Messen seiner Größe und/oder Form durch Erhalten der Haupt- und der Nebenachse der approximierenden Ellipse zu messen; und
wobei die Glättungseinrichtung dazu ausgelegt ist, den Glättungsvorgang mittels eines Gaußfilterungsvorgangs unter Verwendung einer zweidimensionalen Gaußfilterfunktion auszuführen und die Gaußfilterfunktion so einzurichten, dass ihre Standardabweichungen in den Richtungen der zwei senkrechten Achsen auf den Längen der Haupt- und der Nebenachse der Ellipse beruht, die die Größe und/oder die Form des Flecks, wie sie von der Fleckmesseinrichtung gemessen wurden, approximiert.

2. Ultraschalldiagnosevorrichtung nach Anspruch 1, wobei die Fleckmesseinrichtung dazu ausgelegt ist, die Größe und/oder die Form des Flecks auf der Grundlage des Ergebnisses einer Anwendung einer dichten Kombinationsmatrix zu messen, die an den Bilddaten der jeweiligen Frames der Bewegtbilder durchgeführt wird.

3. Ultraschalldiagnosevorrichtung nach Anspruch 1, umfassend: eine Parametermesseinrichtung zum Messen eines Parameters, der die Form des sich bewegenden Bereichs auf der Grundlage der Konturen der jeweiligen Bereiche in jedem Frame der Bewegtbilder darstellt; und
eine Anzeigeeinrichtung (15) zum Anzeigen zeitlicher Variation des Parameters.

4. Ultraschalldiagnosevorrichtung nach Anspruch 1, wobei:
der sich bewegende Bereich das Herz eines zu untersuchenden Objektes ist und
die Kontur eine Kontur einer inneren Membran der linken Herzkammer, einer äußeren Membran der linken Herzkammer, des linken Vorhofs, einer inneren Membran der rechten Herzkammer, einer äußeren Membran der rechten Herzkammer und des rechten Vorhofs des Herzen ist.

5. Ultraschalldiagnosevorrichtung nach Anspruch 4, umfassend:
eine Einrichtung zum Justieren der jeweiligen Konturen, so dass jedes Paar von Klappenringen jeweils ein Verbindungspunkt einer inneren Membran einer linken Herzkammer, einer äußeren Membran der linken Herzkammer und eines linken Vorhofs oder der inneren Membran einer rechten Herzkammer, der äußeren Membran der rechten Herzkammer und eines rechten Vorhofs des Herzen wird.

6. Ultraschalldiagnosevorrichtung nach Anspruch 3, wobei der Parameter das Volumen oder die Achsenlänge von vier Herzkammern, die Entfernung zwischen den Membranen, durch die das Herz gebildet wird, oder die Dicke von Herzmuskeln ist.

7. Ultraschalldiagnosevorrichtung nach Anspruch 3, wobei die Messeinrichtung dazu ausgelegt ist, Parameter mittels des Simpson-Verfahrens zu erhalten.

8. Ultraschalldiagnosevorrichtung nach Anspruch 1, umfassend:
eine Einrichtung (15) zum Anzeigen zeitlicher Variation der Kontur durch dreidimensionales Anordnen der Querschnitte des sich bewegenden Bereichs.

9. Ultraschalldiagnosevorrichtung nach Anspruch 1, umfassend:
eine Einrichtung (15) zum Anzeigen zeitlicher Variation der Kontur durch zeitliches Variieren eines dreidimensionalen Bildes der Kontur des sich bewegenden Bereichs.

10. Ultraschall-Bildverarbeitungsverfahren, umfassend die folgenden Schritte:
1) Übertragen/Empfangen von Ultraschallwellen an/von einem Objekt und Abbilden eines sich bewegenden Objekts mit Bewegtbildern,
2) Messen (22a, 31a), bezüglich jedes Frames der Bewegtbilder, einer Größe und/oder einer Form eines auf jedem Frame erscheinenden Flecks; und
3) Ausführen (22b, 31b) eines Glättungsvorgangs an den Bilddaten jedes Frames gemäß der gemessenen Größe und/oder Form des Flecks;
wobei im Schritt 2) die Größe und/oder die Form des Flecks durch Approximieren des Flecks mit einer Ellipse mit einer Hauptachse und einer Nebenachse und durch Messen seiner Größe und/oder Form durch Erhalten der Haupt- und der Nebenachse der approximierenden Ellipse gemessen wird; und
wobei im Schritt 3) der Glättungsvorgang durch einen Gaußfilterungsvorgang unter Verwendung einer zweidimensionalen Gaußfilterfunktion ausgeführt wird, die so eingerichtet ist, dass ihre Standardabweichungen in den zwei senkrechten Achsen auf den Längen der Haupt- und der Nebenachse der Ellipse beruhen, die die Größe und/oder die Form des Flecks, wie sie von der Fleckmesseinrichtung gemessen wurden, approximiert.

11. Ultraschall-Bildverarbeitungsverfahren nach Anspruch 10, umfassend:
4) Extrahieren einer Kontur des sich bewegenden Bereichs bezüglich eines beliebigen Frames der Bewegtbilder und
5) Detektieren von Bewegung der Kontur bezüglich eines anderen Frames der Bewegtbilder.

12. Ultraschall-Bildverarbeitungsverfahren nach Anspruch 10, wobei im Schritt 2) die Größe und/oder die Form des Flecks durch Anwenden einer dichten Kombinationsmatrix auf die Bilddaten in jedem Frame detektiert wird.

13. Ultraschall-Bildverarbeitungsverfahren nach Anspruch 10, wobei die Kontur eine Kontur einer inneren Membran einer linken Herzkammer, einer äußeren Membran der linken Herzkammer, des linken Vorhofs, einer inneren Membran einer rechten Herzkammer, einer äußeren Membran der rechten Herzkammer und des rechten Vorhofs eines Herzen ist.

14. Ultraschall-Bildverarbeitungsverfahren nach Anspruch 13, wobei Schritt 2) umfasst:
6) Eingeben (23) von Konturpunkten einer inneren Membran einer linken Herzkammer, einer äußeren Membran der rechten Herzkammer, und des linken Vorhofs eines Herzens auf jedem Frame der Bewegtbilder mittels einer Eingabeeinrichtung;
7) Justieren der Konturpunkte, um einen Abschnitt zu erstellen, bei dem Konturpunkte der inneren Membran der linken Herzkammer, der äußeren Membran der linken Herzkammer und des linken Vorhofs des Herzens an einem Klappenring verbunden werden;
8) Herleiten (25) einer Konturlinie durch glattes Verbinden der Konturpunkte und
9) Korrigieren (26) der Herleitung der Konturlinie.

15. Ultraschall-Bildverarbeitungsverfahren nach Anspruch 11, ferner umfassend:
10) Detektieren, wie sich die als eine Konturlinie im Schritt 9) hergeleitete Kontur in jedem Frame der Bewegtbilder bewegt;
11) Berechnen eines Parameters, der sich auf die Bewegung des sich bewegenden Bereichs bezieht, der der Bewegung der Kontur entspricht; und
12) Anzeigen zeitlicher Variation des Parameters.

16. Ultraschall-Bildverarbeitungsverfahren nach Anspruch 11, umfassend:
13) Durchführen von Signalverarbeitung zum geeigneten Anzeigen der Bewegung der Kontur und
14) Anzeigen der Bewegung der Kontur auf der Grundlage des durch die Signalverarbeitung erhaltenen Ergebnisses.

17. Ultraschall-Bildverarbeitungsverfahren nach Anspruch 16, wobei zeitliche Variation der Kontur im Schritt 14) durch dreidimensional angeordnete Querschnitte des sich bewegenden Bereichs angezeigt wird.

18. Ultraschall-Bildverarbeitungsverfahren nach Anspruch 16, wobei zeitliche Variation der Kontur im Schritt 14) durch zeitliches Variieren des dreidimensionalen Bildes einer Kontur der Bewegtbilder angezeigt wird.

## Revendications

1. Appareil de diagnostic ultrasonore comprenant des moyens pour émettre/recevoir des ondes ultrasonores vers/depuis un objet à examiner et former l'image d'un objet mobile avec des images animées, comprenant :
des moyens de mesure de chatoiement, relativement à chaque image des images animées, pour mesurer une taille et/ou une forme d'un chatoiement apparaissant sur chaque image ; et
des moyens de lissage pour effectuer un traitement de lissage sur des données d'image de chaque image en fonction de la taille et/ou de la forme mesurées du chatoiement ;
dans lequel les moyens de mesure de chatoiement sont conçus pour mesurer la taille et/ou la forme du chatoiement par approximation du chatoiement par une ellipse ayant un grand axe et un petit axe et mesure de sa taille et/ou de sa forme par obtention des grand et petit axes de l'ellipse d'approximation ; et
dans lequel les moyens de lissage sont conçus pour effectuer le traitement de lissage par un processus de filtrage gaussien utilisant une fonction de filtrage gaussien bidimensionnel et pour configurer la fonction de filtrage gaussien de manière à ce que ses écarts types dans les directions des deux axes orthogonaux soient basés sur les longueurs du grand axe et du petit axe de l'ellipse approchant la taille et/ou la forme du chatoiement mesurées par les moyens de mesure de chatoiement.

2. Appareil de diagnostic ultrasonore selon la revendication 1, dans lequel les moyens de mesure de chatoiement sont conçus pour mesurer la taille et/ou la forme du chatoiement sur la base du résultat de l'application d'une matrice de cooccurrence de densité sur des données d'image des images respectives de l'image animée.

3. Appareil de diagnostic ultrasonore selon la revendication 1, comprenant :
des moyens de mesure de paramètre pour mesurer un paramètre représentant la forme de la région mobile sur la base du contour des régions respectives dans chaque image de l'image animée ; et
des moyens d'affichage (15) pour visualiser la variation temporelle du paramètre.

4. Appareil de diagnostic ultrasonore selon la revendication 1, dans lequel :
la région mobile est le coeur d'un objet à examiner ; et
le contour est un contour de la membrane interne du ventricule gauche, de la membrane externe du ventricule gauche, de l'oreillette gauche, de la membrane interne du ventricule droit, de la membrane externe du ventricule droit et de l'oreillette droite du coeur.

5. Appareil de diagnostic ultrasonore selon la revendication 4, comprenant :
des moyens pour ajuster les contours respectifs de manière à ce que chaque paire d'anneaux valvulaires devienne un point de réunion de la membrane interne du ventricule gauche, de la membrane externe du ventricule gauche et de l'oreillette gauche, ou de la membrane interne du ventricule droit, de la membrane externe du ventricule droit et de l'oreillette droite du coeur, respectivement.

6. Appareil de diagnostic ultrasonore selon la revendication 3, dans lequel le paramètre est le volume ou la longueur axiale des quatre cavités cardiaques, la distance entre les membranes par lesquelles le coeur est formé, ou l'épaisseur du muscle cardiaque.

7. Appareil de diagnostic ultrasonore selon la revendication 3, dans lequel les moyens de mesure sont conçus pour obtenir des paramètres à l'aide de la méthode de Simpson.

8. Appareil de diagnostic ultrasonore selon la revendication 1, comprenant :
des moyens (15) pour visualiser la variation temporelle du contour par alignement, d'une manière tridimensionnelle, des coupes de la région mobile.

9. Appareil de diagnostic ultrasonore selon la revendication 1, comprenant :
des moyens (15) pour visualiser la variation temporelle du contour par modification temporelle d'une image tridimensionnelle du contour de la région mobile.

10. Procédé de traitement d'image ultrasonore, comprenant :
1) une étape d'émission/réception d'ondes ultrasonores vers/depuis un objet et de formation d'image d'un objet mobile avec des images animées ;
2) une étape (22a, 31a), relativement à chaque image de l'image animée, de mesure d'une taille et/ou d'une forme d'un chatoiement apparaissant sur chaque image ; et
3) une étape (22b, 31b) de réalisation d'un traitement de lissage sur des données d'image de chaque image en fonction de la taille et/ou de la forme mesurées du chatoiement ;
dans lequel, à l'étape 2), la taille et/ou la forme du chatoiement sont mesurées par approximation du chatoiement par une ellipse ayant un grand axe et un petit axe et mesure de sa taille et/ou de sa forme par obtention des grand et petit axes de l'ellipse d'approximation ; et
dans lequel, à l'étape 3), le traitement de lissage est effectué par un processus de filtrage gaussien utilisant une fonction de filtrage gaussien bidimensionnel configurée de manière à ce que ses écarts types dans les directions des deux axes orthogonaux soient basés sur les longueurs du grand axe et du petit axe de l'ellipse approchant la taille et/ou la forme du chatoiement mesurées par les moyens de mesure de chatoiement.

11. Procédé de traitement d'image ultrasonore selon la revendication 10, comprenant :
4) une étape d'extraction d'un contour de la région mobile relativement à une image arbitraire de l'image animée ; et
5) une étape de détection d'un mouvement du contour relativement à une autre image de l'image animée.

12. Procédé de traitement d'image ultrasonore selon la revendication 10, dans lequel, à l'étape 2), la taille et/ou la forme du chatoiement sont détectées par application d'une matrice de cooccurrence de densité sur des données d'image de chaque image.

13. Procédé de traitement d'image ultrasonore selon la revendication 10, dans lequel le contour est un contour de la membrane interne du ventricule gauche, de la membrane externe du ventricule gauche, de l'oreillette gauche, de la membrane interne du ventricule droit, de la membrane externe du ventricule droit et de l'oreillette droite d'un coeur.

14. Procédé de traitement d'image ultrasonore selon la revendication 13, dans lequel l'étape 2) comprend :
6) une étape (23) de saisie de points de contour de la membrane interne du ventricule gauche, de la membrane externe du ventricule gauche et de l'oreillette gauche du coeur sur une image de l'image animée à l'aide d'un moyen d'entrée ;
7) une étape d'ajustement des points de contour afin de former une partie au niveau de laquelle des points de contour de la membrane interne du ventricule gauche, de la membrane externe du ventricule gauche et de l'oreillette gauche du coeur se rejoignent au niveau d'un anneau valvulaire ;
8) une étape (25) d'obtention d'une ligne de contour par liaison lissée des points de contour ; et
9) une étape (26) de correction de l'obtention de la ligne de contour.

15. Procédé de traitement d'image ultrasonore selon la revendication 11, comprenant en outre :
10) une étape consistant à détecter comment le contour obtenu sous la forme d'une ligne de contour à l'étape 9) se déplace dans chaque image de l'image animée ;
11) une étape de calcul d'un paramètre relatif au mouvement de la région mobile correspondant au mouvement du contour ; et
12) une étape de visualisation de la variation temporelle du paramètre.

16. Procédé de traitement d'image ultrasonore selon la revendication 11, comprenant :
13) une étape de réalisation d'un traitement de signal pour visualiser d'une manière appropriée un mouvement du contour ; et
14) une étape de visualisation du mouvement du contour sur la base du résultat obtenu par le traitement de signal.

17. Procédé de traitement d'image ultrasonore selon la revendication 16, dans lequel la variation temporelle du contour, à l'étape 14), est visualisée par des coupes alignées d'une manière tridimensionnelle de la région mobile.

18. Procédé de traitement d'image ultrasonore selon la revendication 16, dans lequel la variation temporelle du contour, à l'étape 14), est visualisée par modification temporelle de l'image tridimensionnelle d'un contour de la région mobile.
